Europäisches Patentamt

European Patent Office

Office européen des brevets

(19)

(11) **EP 1 161 218 B1**

(12) **FASCICULE DE BREVET EUROPEEN**

(45) Date de publication et mention
de la délivrance du brevet:
**27.10.2004 Bulletin 2004/44**

(21) Numéro de dépôt: **00910934.9**

(22) Date de dépôt: **14.03.2000**

(51) Int Cl.⁷: **A61J 3/10**

(86) Numéro de dépôt international:
**PCT/FR2000/000609**

(87) Numéro de publication internationale:
**WO 2000/054725 (21.09.2000 Gazette 2000/38)**

(54) **PROCEDE DE FABRICATION DE COMPRIMES PHARMACEUTIQUES PAR COMPRESSION**

VERFAHREN ZUR HERSTELLUNG VON PHARMAZEUTISCHEN TABLETTEN DURCH PRESSUNG

METHOD FOR MAKING PHARMACEUTICAL TABLETS BY COMPRESSION

(84) Etats contractants désignés:
**AT BE CH CY DE DK ES FI FR GB GR IE IT LI LU MC NL PT SE**

(30) Priorité: **16.03.1999 FR 9903230**

(43) Date de publication de la demande:
**12.12.2001 Bulletin 2001/50**

(73) Titulaire: **TALC DE LUZENAC**
**09250 Luzenac (FR)**

(72) Inventeurs:
• **ARSEGUEL, Didier**
**F-31450 Deyme (FR)**

• **BAEZA, Richard**
**F-31100 Toulouse (FR)**
• **RODRIGUEZ, Fernand**
**F-31320 Aureville (FR)**
• **TORTOSA, Florence**
**F-31860 Labarthe sur Leze (FR)**

(74) Mandataire:
**Cabinet BARRE LAFORGUE & associés**
**95, rue des Amidonniers**
**31000 Toulouse (FR)**

(56) Documents cités:
**EP-A- 0 667 147**      **US-A- 5 376 382**

## Description

L'invention concerne un procédé de fabrication de comprimés pharmaceutiques par compression d'une poudre de base, généralement composée majoritairement d'au moins un liant (celluloses, lactoses, phosphates...), d'un désintégrant (amidon,...) et d'un ou plusieurs principes actifs comme est connu du EP-A-667 147. Par "compression", on entend le procédé classique, avec ou sans granulation préalable, consistant à amener la poudre de base dans un moule, à comprimer celle-ci au moyen d'un poinçon de compression, puis à éjecter le comprimé obtenu au moyen d'un poinçon d'éjection.

Tous les procédés classiques de fabrication de comprimés (procédé de compression directe et procédé avec granulation préalable) posent un problème d'écoulement de la poudre qui, à l'heure actuelle, est résolu soit par addition d'un régulateur d'écoulement (terme anglais : "glidant") qui peut être de la silice ou du talc, soit par une sélection appropriée des constituants de la poudre de base (liant, désintégrant). Dans cette fonction de régulation d'écoulement, le talc classiquement ajouté à la poudre de base donne de bons résultats consistant à favoriser un écoulement naturel, uniforme et régulier dans la poudre sans prise en masse. On pourra notamment se reporter à cet égard à la publication suivante : "Shabbir Dawoodbhai and Christopher T. Rhodes, Drug. Dev. Ind. Pharm. 16(16), 2409-2429 (1990)".

Le procédé de compression pose un autre problème de fabrication lors de la mise en oeuvre de la compression. Cette opération étant réalisée à très haute cadence (de l'ordre notamment de 250 000 comprimés par heure pour les gros comprimés et 600 000 à 700 000 comprimés par heure pour les plus petits), il est nécessaire que la poudre à comprimer soit parfaitement lubrifiée pour éviter un grippage et un échauffement des outils de presse ainsi qu'un laminage des particules de la poudre pouvant conduire à un risque de dégradation de celle-ci. A cet effet, préalablement à la compression, on ajoute à la poudre de base un ou des lubrifiants (terme anglais : "lubricant") qui ont pour fonction d'assurer une lubrification dans cette opération mécanique de compression. Cette lubrification mécanique se différencie fondamentalement de la régulation d'écoulement préalablement évoquée compte tenu des conditions totalement différentes des deux opérations. Il a été constaté que le talc qui donne satisfaction en tant que régulateur d'écoulement ne donne pas de résultats satisfaisants en tant que lubrifiant mécanique dans l'opération de compression. On pourra se reporter aux publications suivantes qui montrent les mauvaises performances du talc en tant que lubrifiant mécanique : "C.J. LEWIS and E. SHOTTON, J. Pharm. Pharmacol. 1965, 17, Suppl. 825-865" ; "Eino NELSON et al, J. Ameri. Pharmaceut. Ass. Vol. XLIII, N° 10, p. 596-602, Octobre 1954" ; "Artalejo Ortega B et al, Cienc. Pharm. 1998, 8(2) p. 59-68".

Ces mauvaises performances du talc comme lubrifiant mécanique ont conduit les fabricants de comprimés pharmaceutiques à ajouter à la poudre de base à comprimer un autre type de lubrifiants, à savoir des lubrifiants organiques tels que stéarate de magnésium, polyéthylène glycol, triglycérides,... On pourra se référer aux publications évoquées ci-dessus qui montrent les bonnes performances de ces lubrifiants organiques comparées à celles du talc. Toutefois, ces lubrifiants organiques présentent de graves inconvénients spécifiques. D'une part ils entraînent une baisse notable des propriétés mécaniques du comprimé (dureté et résistance à la rupture) et celui-ci a une tendance à se casser ; d'autre part, ces lubrifiants organiques peuvent interférer avec le ou les principes actifs et réduire sa biodisponibilité en modifiant les cinétiques de dissolution (en particulier par enrobage total ou partiel). Enfin, il faut noter que ces lubrifiants organiques sont généralement coûteux et augmentent le coût des comprimés obtenus.

A la connaissance des inventeurs, le talc seul n'a jamais pu être utilisé en tant que lubrifiant mécanique en raison de ses performances médiocres comme indiqué ci-dessus. Toutefois, on a proposé d'utiliser des combinaisons lubrifiant organique/talc. On a pu constater que la combinaison du talc et d'un lubrifiant organique a des effets complexes et que, dans certains cas, elle réduit les performances de lubrification tandis qu'elle augmente celles-ci dans d'autres cas. On se reportera à la publication suivante qui étudie les résultats obtenus par cette combinaison talc/ lubrifiant organique : "Birgit MECHTERSHEIMER and Heinz SUCKER, Pharmaceutical Techn. Fev. 1986, p. 38-50". Cette publication conclue que, si la proportion de stéarate de magnésium est suffisante, le talc a un effet défavorable, mais que, si cette proportion est trop faible, le talc peut avoir un effet favorable ; il est dit dans cette publication que, du fait des effets négatifs du stéarate de magnésium sur les comprimés (friabilité, dureté et temps de désagrégation), l'addition de talc, plutôt que l'augmentation de la concentration de stéarate, paraît être la meilleure option pour améliorer la formation du comprimé. Toutefois, les essais réalisés montrent que l'amélioration de la lubrification mécanique ainsi obtenue par la combinaison talc/lubrifiant organique est bonne en valeur relative mais très insuffisante en valeur absolue : les courbes de la figure 10 (p. 46 de la publication sus-évoquée) montrent par exemple :

- que pour une proportion de 0,1 % de stéarate, la présence de talc permet d'abaisser la force de grippage de 1400 N environ à 900 N environ (avec 5 % de talc),
- mais que, quelle que soit la proportion de stéarate, la force de grippage reste, en présence de talc, très supérieure à ce qu'elle est en utilisant 1 % de stéarate en l'absence de talc (force de grippage de l'ordre de 650 N en l'absence de talc).

Or, pour fabriquer des comprimés à très haute cadence, la qualité de la lubrification mécanique est essentielle et l'on ne peut se contenter d'un facteur de réduction des forces de grippage de 2 à 2,5 comme cela est obtenu dans le meilleur des cas en utilisant la combinaison talc/stéarate de magnésium. On estime généralement, pour les comprimés habituels fabriqués dans le domaine pharmaceutique, qu'il ne faut pas dépasser des forces de grippage résiduelles de l'ordre de 500 Newtons sous peine de problèmes de production.

Dans ces conditions, on conçoit que l'intérêt de la combinaison talc/lubrifiant organique n'existe que dans certains cas très particuliers et que, de toute façon, l'emploi du talc (qui permet de réduire les inconvénients spécifiques du lubrifiant organique) se fait au détriment de la lubrification mécanique qui devient moins bonne en présence de talc.

La présente invention se propose de réaliser une lubrification mécanique satisfaisante de l'opération de compression dans la fabrication de comprimés pharmaceutiques (autorisant des cadences élevées sans échauffement excessif ni grippage), tout en permettant de réduire de façon significative la proportion de lubrifiant organique mélangé à la poudre de base, voire de supprimer totalement ce lubrifiant organique.

Un des objectifs de l'invention est ainsi de limiter, voire de supprimer, les effets négatifs spécifiques des lubrifiants organiques sur les comprimés (dureté et résistance à la rupture du comprimé obtenu, réduction de la biodisponibilité du principe actif).

Un autre objectif est de réduire le coût de formulation des poudres par la réduction des quantités de lubrifiants organiques.

A cet effet, le procédé de fabrication de comprimés pharmaceutiques par compression d'une poudre est caractérisé, selon la présente invention, en ce que, préalablement à la compression, on ajoute à la poudre de base, du talc présentant une répartition granulométrique telle que le diamètre moyen $D_{50}$ soit inférieur ou égal à 4,5 microns.

On entend par "talc" soit le minéral silicate de magnésium hydraté, soit le minéral chlorite (silicate de magnésium et d'aluminium hydraté), soit un mélange des deux, associé le cas échéant à d'autres minéraux (dolomite,...), soit encore, une substance issue du talc, présentant des propriétés analogues.

De préférence, on utilise du talc ayant un diamètre de coupure $D_{95}$ inférieur ou égal à 15 microns.

Par "diamètre moyen $D_{50}$", on entend un diamètre tel que 50 % des particules en poids ont une taille inférieure audit diamètre ; par "diamètre de coupure $D_{95}$", on entend un diamètre tel que 95 % des particules en poids ont une taille inférieure audit diamètre. Pour des particules non sphériques, la taille est constituée par le diamètre sphérique équivalent (diamètre de Stokes). Toutes les mesures des diamètres $D_{50}$ et $D_{95}$ sont effectuées au moyen d'un appareil "Sédigraph" (marque déposée) par sédimentation par gravité conformément à la norme AFNOR X11-683.

Les expérimentations ont permis de constater de façon surprenante que, si au lieu d'utiliser un talc standard comme c'est le cas dans les essais de l'art antérieur ($D_{50}$ de l'ordre de 8 à 15 microns), on utilise, mélangé à la poudre de base, un talc de granulométrie fine ou très fine, on obtient un effet de lubrification mécanique significativement supérieur, et ce dans tous les cas, permettant, soit de supprimer le lubrifiant organique, soit d'en réduire la proportion de façon significative ; la lubrification ainsi obtenue autorise une production à haute cadence dans de bonnes conditions. Il convient de souligner que cet effet remarquable est inattendu, car en diminuant la granulométrie du talc, l'on pourrait s'attendre à un effet inverse dû à une agglomération de la poudre par les petites particules de talc, conduisant à des risques de prise en masse. Aucune explication n'a pu être fournie actuellement.

Par ailleurs, on a pu constater que, dans les cas difficiles (poudre de base ayant une tendance marquée au grippage, ou très forte cadence de production), il est avantageux d'utiliser un talc de type sphéroïde, présentant sensiblement une masse volumique apparente tassée supérieure à 0,15 g/cm$^3$, et avantageusement sensiblement comprise entre 0,15 et 0,55 g/cm$^3$, en particulier talc microcristallin. La masse volumique apparente tassée d'une poudre est définie par la norme AFNOR NFT30-042. Pour un talc, elle donne une image de la forme des grains : en effet, à répartition granulométrique équivalente, un talc ayant des grains de forme lamellaire très allongée possède une masse volumique apparente tassée plus faible que celle d'un talc ayant des grains de forme moins allongée (plus sphéroïde). Ainsi, à granulométrie équivalente, ce paramètre peut être considéré en pratique pour le talc comme un coefficient permettant d'apprécier la forme des particules de celui-ci. La lubrification mécanique est significativement meilleure avec les talcs sus-évoqués de type sphéroïde, sans que l'explication de ce fait soit évidente à donner.

La proportion de talc ajouté dépend du cas d'espèce et de la nature de la poudre de base. Cette proportion pondérale est en général comprise sensiblement entre 1 % et 6 % par rapport à la poudre de base, pour les formulations habituelles utilisées (liant de groupe : celluloses, lactoses, phosphates...; diamètre moyen $D_{50}$ des particules sensiblement compris entre 50 microns et 250 microns).

Selon un mode de mise en oeuvre optimum du procédé, on ajoute à la poudre de base, du talc ayant les caractéristiques suivantes :

- répartition granulométrique telle que le diamètre moyen $D_{50}$ soit sensiblement compris entre 0,5 microns et 2,5 microns,
- masse volumique apparente tassée sensiblement comprise entre 0,15 g/cm$^3$ et 0,40 g/cm$^3$.

Ce choix du talc ajouté permet dans de nombreux cas de n'utiliser que le talc comme lubrifiant mécanique. Dans les autres cas, un autre lubrifiant pourra être ajouté à la poudre pour accroître encore l'effet de lubrification :

lubrifiant organique tel que stéarate de magnésium, ou même lubrifiant siliconé. Les proportions pondérales de ce lubrifiant additionnel sont réduites par rapport aux proportions classiques utilisées et, en tous cas, inférieures à la proportion de talc ajoutée.

Les performances de lubrification obtenues sont généralement mesurées par l'indice de lubrification $\frac{f}{F}$ (rapport de la force — f- transmise au poinçon inférieur par la force de compression — F- appliquée au poinçon supérieur), la force d'éjection (force appliquée par le poinçon inférieur pour produire l'éjection du comprimé) et par la force de grippage résiduelle (force résiduelle appliquée par le comprimé au poinçon inférieur lorsque le poinçon supérieur n'exerce plus aucune force sur le comprimé).

Les exemples qui suivent illustrent le procédé de l'invention et les performances atteintes mesurées par les 3 paramètres ci-dessus indiqués.

La poudre de base mise en oeuvre dans ces exemples est un mélange ayant les caractéristiques suivantes.

| Produits | % (proportion pondérale) |
|---|---|
| Liant 1 Cellulose micro-cristalline "Avicel pH 101" (marque déposée) | 40 |
| Liant 2 Lactose "Fast flo" (marque déposée) | 45 |
| Désintégrant Amidon "Sepistab ST200" (marque déposée) | 15 |

Les mesures sont réalisées à l'aide d'une machine à comprimer alternative instrumentée en force et en déplacement, et équipée d'un jeu de poinçons cylindriques de 1 cm$^2$ de surface.

Les réglages de la machine (volume de la chambre de compression réglé par le déplacement du poinçon inférieur) sont effectués avec la formule de base pour obtenir un comprimé de masse moyenne de 350 mg. Ce volume sera maintenu constant pendant tous les essais afin de noter les variations de masse éventuelles des comprimés formés.

La force de compression F, déterminée par le réglage du poinçon supérieur, est constante pour toutes les formulations et est fixée à environ 1500 DaN (1500 kg/cm$^2$ de surface).

EXEMPLE 1 : Effet de lubrification mécanique

Cet exemple fournit un comparatif entre la poudre de base, la poudre de base additionnée de 3% (proportion pondérale) de talc A conforme à l'invention et la poudre de base additionnée de 3% (proportion pondérale) de talc standards B ou C, non conformes à l'invention.

| Répartition granulométrique des talcs : | | | |
|---|---|---|---|
| Talcs | D50 (µm) | D95 (µm) | Commentaires |
| A | 0.62 | 2.5 | Conforme à l'invention |
| B | 15 | 40 | Standard |
| C | 10 | 29 | Standard |

| Résultats : (moyenne obtenue sur 10 cycles de compression) | | | |
|---|---|---|---|
| Paramètres de lubrification mécanique | Poudre de base | Poudre de base + talc A | Poudre de base + talc B | Poudre de base + talc C |
| Indice de lubrification | 0.63 (± 0.1) | 0.79 (± 0.1) | 0.69 (± 0.1) | 0.70 (± 0.1) |
| Force de grippage . résiduelle (DaN) | 128 (± 6.0) | 40 (± 0.0) | 83 (± 4.0) | 77 (± 4.0) |
| Force d'éjection | 233 (± 10.0) | 51 (± 0.0) | 87 (± 5.0) | 87 (± 5.0) |

On constate que le talc A conforme à l'invention donne un résultat satisfaisant en terme de lubrification mécanique et significativement supérieur aux talcs standards, et ce sans autre lubrifiant.

EXEMPLE 2 : Influence de la répartition granulométrique

Cet exemple donne une comparaison entre la poudre de base et la poudre de base additionnée de 3 % (proportion pondérale) des talcs D ou E, conformes à l'invention, de répartition granulométrique différente.

| Répartition granulométrique des talcs | | |
|---|---|---|
| Talcs | D50 (µm) | D95 (µm) |
| D | 1.6 | 4.2 |
| E | 3.3 | 9.6 |

| Résultats : (moyenne de 10 cycles de compression) | | | |
|---|---|---|---|
| Paramètres de lubrification mécanique | Poudre de base | Poudre de base + talc D | Poudre de base + talc E |
| Indice de lubrification | 0.60 ($\pm$ 0.1) | 0.78 ($\pm$ 0.1) | 0.76 ($\pm$ 0.1) |
| Force de grippage résiduelle (DaN) | 98 ($\pm$ 3.6) | 23 ($\pm$ 0.0) | 40 ($\pm$ 2) |
| Force d'éjection | 204 ($\pm$ 30.0) | 37 ($\pm$ 0.0) | 50 ($\pm$ 2) |

(les valeurs différentes pour la poudre de base entre cet exemple 2 et l'exemple 1 sont dues à l'utilisation de lots différents).

On constate que les talcs D et E conformes à l'invention donnent un résultat satisfaisant en terme de lubrification mécanique.

EXEMPLE 3 : Influence de la forme des grains de talc

Cet exemple donne un comparatif entre la poudre de base, la poudre de base additionnée de 1 % (proportion pondérale) de talc G conforme à l'invention et la poudre de base additionnée de 1 % (proportion pondérale) de talc H conforme à l'invention, mais moins sphérique ayant des grains de forme plus allongée que le talc G.

| Répartition granulométrique et masse volumique apparente tassée des talcs | | | |
|---|---|---|---|
| Talcs | D50 (µm) | D95 (µm) | Masse volumique apparente tassée (g/cm$^3$) |
| G | 0.9 | 2.5 | 0.24 |
| H | 0.9 | 3.0 | 0.22 |

| Résultats : (moyenne de 10 cycles de compression) | | | |
|---|---|---|---|
| Paramètres de lubrification mécanique | Poudre de base | Poudre de base + talc G | Poudre de base + talc H |
| Indice de lubrification | 0.63 ($\pm$ 0.1) | 0.75 ($\pm$ 0.1) | 0.72 ($\pm$ 0.1) |

(dans cet exemple, seul l'indice de lubrification a été mesuré).

On constate que les deux talcs G et H conformes à l'invention donnent un résultat satisfaisant en terme de lubrification mécanique, toutefois le talc H, moins sphérique, donne un résultat inférieur au talc G.

EXEMPLE 4 : Combinaison talc - lubrifiant organique (stéarate de magnésium)

Cet exemple donne un comparatif en terme de lubrification mécanique et dureté des comprimés entre la poudre de base, la poudre de base additionnée de 1% ou 0,2% (proportion pondérale) de stéarate de magnésium et la poudre de base additionnée de la combinaison 0,2% de stéarate de magnésium et 3% (proportion pondérale) de talc D conforme à l'invention.

**Mesure de dureté**

La dureté ou résistance à la rupture est mesurée à l'aide de l'appareil pharmacopée ("pharmatest" référence pTB311) sur 10 comprimés prélevés aléatoirement.

**Répartition granulométrique du talc D (voir exemple 2)**

| Résultats : (moyenne de 10 cycles de compression) | | | | |
|---|---|---|---|---|
| Paramètres de lubrification mécanique | Poudre de base | Poudre de base +1% stéarate de Mg | Poudre de base + 0,2% stéarate de Mg | Poudre de base + 0,2% de stéarate de Mg + 3% talc D |
| Indice de lubrification Force de grippage résiduelle (DaN) Force d'éjection (DaN) | 0,6 (±0,1) 92 (±20,0) 201 (±70,0) | 0,89 (±0,1) 3,4 (±0,0) 13,4 (±0,0) | Résultats incohérents Lubrification très mauvaise | 0,8 (±0,1) 19 (±1,5) 26(±1,5) |
| Dureté des comprimés (N) | 112 (±4) | 47 (±2,0) | Non mesurable (coefficient de variation supérieur à 5%) | 87 (±1,6) |

On constate que l'addition de talc D conforme à l'invention permet de réduire le taux de lubrifiant mécanique (stéarate de magnésium) de 80% (à ce taux, le stéarate de magnésium seul ne donne pas un résultat satisfaisant : très mauvaise lubrification mécanique), tout en maintenant une lubrification mécanique très satisfaisante et en augmentant la dureté des comprimés.

EXEMPLE 5 : Combinaison talc-silicone

Cet exemple donne un comparatif de la poudre de base, de la poudre de base additionnée de 1% ou 0,4% (proportion pondérale) de silicone et de la poudre de base additionnée de la combinaison de 0,4% de silicone et de 1% (proportion pondérale) de talc D conforme à l'invention.

Caractéristiques du silicone

Polydiméthylsiloxane de viscosité 50 cps "DC 200 50 cps"

Répartition granulométrique du talc (voir exemple 2)

| Résultats : (moyenne de 10 cycles de compression) | | | | |
|---|---|---|---|---|
| Paramètres de lubrification mécanique | Poudre de base | Poudre de base +1% de silicone | Poudre de base + 0,4% de silicone | Poudre de base + 0,4% de silicone et 1% de talc D |
| Indice de lubrification Force de grippage résiduelle (DaN) Force d'éjection (DaN) | 0,6 (±0,1) 98,1(±3,6) 204 (±30,0) | 0,82 (±0.1) 17 (±0,0) 20 (±0,0) | 0,74 (±0,1) 46 (±9,0) 47 (±8,0) | 0,81 (±0,1) 13 (±0,0) 33(±0,0) |
| Dureté des comprimés (N) | 112 (±4) | 55 (±7,0) | 63 (±8,0) | 51 (±4) |

On constate que l'addition de talc D conforme à l'invention permet d'augmenter significativement la lubrification mécanique, de réduire le taux de silicone (-60%), tout en maintenant une dureté acceptable.

La combinaison talc D à 1% / silicone à 0,4% est quasiment équivalente à la combinaison poudre de base avec ±1% de silicone, mais à un coût de la formulation fortement réduit (prix moyen du silicone : 50 F/kg).

**Revendications**

1. Procédé de fabrication de comprimés pharmaceutiques par compression d'une poudre, **caractérisé en ce que**, préalablement à la compression, on ajoute à la poudre de base, du talc présentant une répartition granulométrique telle que le diamètre moyen $D_{50}$ soit inférieur ou égal à 4,5 microns.

2. Procédé selon la revendication 1, **caractérisé en ce que** l'on ajoute à la poudre de base, du talc présentant une répartition granulométrique telle que le diamètre moyen $D_{50}$ soit inférieur ou égal à 4,5 microns et que le diamètre de coupure $D_{95}$ soit inférieur ou égal à 15 microns.

3. Procédé selon l'une des revendications 1 ou 2, **caractérisé en ce que** l'on ajoute à la poudre de base, du talc présentant une masse volumique apparente tassée sensiblement comprise entre 0,15 et 0,55 g/cm$^3$.

4. Procédé selon l'une des revendications 1, 2 ou 3, **caractérisé en ce que** l'on ajoute à la poudre de base, du talc ayant les caractéristiques suivantes :

   - répartition granulométrique telle que le diamètre moyen $D_{50}$ soit sensiblement compris entre 0,5 microns et 2,5 microns,
   - masse volumique apparente tassée sensiblement comprise entre 0,15 g/cm$^3$ et 0,40 g/cm$^3$.

5. Procédé selon l'une des revendications 1, 2, 3 ou 4, **caractérisé en ce que** l'on ajoute une proportion pondérale de talc sensiblement comprise entre 1 % et 6 % par rapport à la poudre de base.

6. Procédé selon l'une des revendications 1 à 5, dans lequel ou ajoute également à la poudre de base au moins un lubrifiant organique, en particulier du stéarate de magnésium.

7. Procédé selon l'une des revendications 1 à 5, dans lequel on ajoute également à la poudre de base un lubrifiant siliconé.

8. Procédé selon l'une des revendications 6 ou 7, **caractérisé en ce que** l'on mélange à la poudre de base le lubrifiant additionnel en proportion pondérale inférieure à la proportion de talc ajouté.

9. Procédé selon l'une des revendications 1 à 8, dans lequel la poudre de base est à base d'un liant du groupe : celluloses, lactoses, phosphates.

10. Procédé selon la revendication 9, dans lequel le liant de la poudre de base présente un diamètre moyen de particules $D_{50}$ sensiblement compris entre 50 microns et 250 microns.

**Patentansprüche**

1. Verfahren zur Herstellung von pharmazeutischen Tabletten durch Komprimieren eines Pulvers, **dadurch gekennzeichnet, dass** dem Basispulver vor dem Komprimieren Talkum mit einer solchen Korngrößenverteilung zugegeben wird, dass der mittlere $D_{50}$-Durchmesser gleich oder kleiner als 4,5 Mikron ist.

2. Verfahren nach Anspruch 1, **dadurch gekennzeichnet, dass** dem Basispulver Talkum mit einer solchen Korngrößenverteilung zugegeben wird, dass der mittlere $D_{50}$-Durchmesser gleich oder kleiner als 4,5 Mikron und der $D_{95}$-Schnittdurchmesser gleich oder kleiner als 15 Mikron ist.

3. Verfahren nach Anspruch 1 oder 2, **dadurch gekennzeichnet, dass** dem Basispulver Talkum mit einer Schüttdichte im Wesentlichen zwischen 0,15 und 0,55 g/cm$^3$ zugegeben wird.

4. Verfahren nach Anspruch 1, 2 oder 3, **dadurch gekennzeichnet, dass** dem Basispulver Talkum mit den folgenden Eigenschaften zugegeben wird:

- eine solche Korngrößenverteilung, das der mittlere $D_{50}$-Durchmesser zwischen 0,5 Mikron und 2,5 Mikron liegt,
- eine Schüttdichte, die im Wesentlichen zwischen 0,15 g/cm$^3$ und 0,40 g/cm$^3$ liegt.

5. Verfahren nach Anspruch 1, 2, 3 oder 4, **dadurch gekennzeichnet, dass** ein Gewichtsanteil an Talkum von im Wesentlichen zwischen 1 und 6 % bezogen auf das Basispulver zugegeben wird.

6. Verfahren nach einem der Ansprüche 1 bis 5, bei dem dem Basispulver auch wenigstens ein organisches Gleitmittel, insbesondere Magnesiumstearat zugegeben wird.

7. Verfahren nach einem der Ansprüche 1 bis 5, bei dem dem Basispulver auch ein Silikongleitmittel zugegeben wird.

8. Verfahren nach Anspruch 6 oder 7, **dadurch gekennzeichnet, dass** dem Basispulver das zusätzliche Gleitmittel in einem Gewichtsanteil beigemengt wird, der geringer ist als der Anteil des zugegebenen Talkums.

9. Verfahren nach einem der Ansprüche 1 bis 8, bei dem das Basispulver auf einem Bindemittel der Gruppe Cellulosen, Lactosen, Phosphate basiert.

10. Verfahren nach Anspruch 9, bei dem das Bindemittel des Basispulvers einen mittleren $D_{50}$-Partikeldurchmesser im Wesentlichen zwischen 50 Mikron und 250 Mikron aufweist.

**Claims**

1. Method for manufacturing pharmaceutical tablets by compressing a powder **characterised in that**, prior to compression, talc having a particle size distribution such that the mean diameter $D_{50}$ is less than or equal to 4.5 microns, is added to the base powder.

2. Method according to claim 1, **characterised in that**, talc having a particle size distribution such that the mean diameter $D_{50}$ is less than or equal to 4.5 microns and the cut-off diameter $D_{95}$ is less than or equal to 15 microns, is added to the base powder.

3. Method according to either of claims 1 or 2, **characterised in that**, talc having a compacted apparent density substantially between 0.15 and 0.55 g/m$^3$, is added to the base powder.

4. Method according to one of claims 1, 2 or 3, **characterised in that**, talc having the following characteristics is added to the base powder:

   - a particle size distribution such that the mean diameter $D_{50}$ lies substantially between 0.5 microns and 2.5 microns,

   - a compacted apparent density substantially between 0.15 g/m$^3$ and 0.40 g/m$^3$.

5. Method according to one of claims 1, 2, 3 or 4, **characterised in that** a proportion of talc is added substantially between 1 % and 6 % by weight with respect to the base powder.

6. Method according to one of claims 1 to 5, wherein at least one organic lubricant, in particular magnesium stearate, is also added to the base powder.

7. Method according to one of claims 1 to 5, wherein a silicone lubricant is also added to the base powder.

8. Method according to either of claims 6 or 7, **characterised in that**, the additional lubricant is added to the base powder in a proportion by weight less than the proportion of talc added.

9. Method according to one of claims 1 to 8, wherein the base powder is based on a binder of the group: celluloses, lactoses and phosphates.

10. Method according to claim 9, wherein the binder for the base powder has a mean particle size diameter $D_{50}$ sub-

stantially between 50 microns and 250 microns.